Europäisches Patentamt

European Patent Office

Office européen des brevets

(19)

(11)  **EP 1 541 196 A1**

(12)  **EUROPEAN PATENT APPLICATION**

(43) Date of publication:
**15.06.2005 Bulletin 2005/24**

(51) Int Cl.⁷: **A61N 5/10**

(21) Application number: **04078362.3**

(22) Date of filing: **10.12.2004**

| (84) Designated Contracting States:<br>**AT BE BG CH CY CZ DE DK EE ES FI FR GB GR HU IE IS IT LI LT LU MC NL PL PT RO SE SI SK TR**<br>Designated Extension States:<br>**AL BA HR LV MK YU**<br><br>(30) Priority: **12.12.2003 US 529124 P**<br>**07.12.2004 US 7117**<br><br>(71) Applicant: **Siemens Medical Solutions USA, Inc.**<br>**Malvern, PA 19355-1406 (US)** | (72) Inventors:<br>• **Moore, Terrence E.**<br>**94523 Pleasant Hill, CA (US)**<br>• **Well, Michael D.**<br>**80524 Port Collins, CO (US)**<br>• **Svatos, Michelle Marie**<br>**94602 Oakland, CA (US)**<br><br>(74) Representative: **Condon, Neil**<br>**Siemens Shared Services,**<br>**c/o Siemens AG,**<br>**Postfach Box 22 16 34**<br>**80506 München (DE)** |

(54)  **Safe combination of radiation treatment and delivery of a treatment agent**

(57)    Embodiments may include determination (S3) of a treatment plan associated with a patient, the treatment plan including a first treatment mode in which a treatment agent is present within a portion of the patient and a second treatment mode in which the portion of the patient is irradiated, determination (S4), independent from the second treatment mode, of whether the first treatment mode meets a first safety threshold, determination (S5), independent from the first treatment mode, of whether the second treatment mode meets a second safety threshold, and determination (S6) of whether a combination of the first treatment mode and the second treatment mode meets a third safety threshold.

FIG. 1

EP 1 541 196 A1

## Description

CROSS REFERENCE TO RELATED APPLICATIONS

This application claims priority to provisional application serial no. 60/529,124, filed on December 12, 2003 and entitled "Modular Coordinating System for Combinatorial Products"; and is related to commonly assigned, copending non-provisional U.S. patent application serial no. 10/965,434 entitled "Radiation Treatment System Utilizing Therapeutic Agent and Associated Identifier", filed on October 13, 2004.

BACKGROUND

Field

Embodiments described herein relate generally to medical treatment using two or more treatment modes.

Description

According to conventional radiation treatment, a beam of radiation is directed toward a tumor located within a patient. The radiation beam delivers a predetermined dose of therapeutic radiation to the tumor according to a treatment plan. The delivered radiation kills cells of the tumor by causing ionizations within the cells.

A kilovoltage radiation treatment system produces a divergent beam of X-ray radiation having energies in the 50 to 150 keV range, and might focus the beam on a target site using a lens designed for this purpose. At these energies, most cellular damage caused by the radiation beam is due to photoelectric absorption. The amount of absorption, and the resulting cellular damage, may be magnified by introducing a dose-enhancing biochemical agent into the target site. Some kilovoltage radiation treatment systems employing this technique are described in U.S. Patent Nos. 6,125,295 and 6,366,801.

Generally, in the kilovoltage energy range, the absorption cross-section of an agent having an atomic weight greater than 50 is often significantly higher than the elements of which most human tissue is composed. Therefore, if a suitable radiation beam irradiates a tissue volume containing such an agent, more photons will be stopped by the volume than would be stopped in the absence of the agent. The resulting tissue damage will be greater than tissue damage that would occur without the element, because most of the increased stoppages will be due to photoelectric absorption. Accordingly, a heavy element-carrying biochemical agent is sometimes used as a dose-enhancing treatment agent.

The dose-enhancing effect of treatment agents can be beneficial, since cure rates for tumors often increase with increased radiation doses. However, it can be difficult to evaluate the safety and/or efficacy of treatments that utilize a combination of treatment modes. Such treatments include not only the above-described treatment but also photodynamic therapy and boron neutron capture therapy, among others. Combination treatments potentially increase patient risk with respect to single-mode treatments due to additional variables and overlapping toxicities.

Even if judged to be safe and effective, combination treatment must still be delivered according to prescribed parameters. For example, delivery of both a radiation beam and a treatment agent to a target site must be executed to ensure that particular tissues experience radiation doses that are specified by an associated treatment plan. Improved control and/or efficiency of such delivery are desired.

SUMMARY

To address at least the foregoing, some embodiments of the present invention provide a system, method, apparatus, and means to determine a treatment plan associated with a patient, the treatment plan including a first treatment mode in which a treatment agent is present within a portion of the patient and a second treatment mode in which the portion of the patient is irradiated, to determine if, independent from the second treatment mode, the first treatment mode meets a first safety threshold, to determine if, independent from the first treatment mode, the second treatment mode meets a second safety threshold, and to determine if a combination of the first treatment mode and the second treatment mode meets a third safety threshold.

According to some embodiments, provided are delivery of treatment to a patient in accordance with a first treatment mode in which a treatment agent is present within a portion of the patient and a second treatment mode in which the portion of the patient is irradiated, determination that the delivered treatment is not proceeding in accordance with a desired treatment outcome, adjustment of the first treatment mode and/or the second treatment mode, and delivery of treatment to the patient in accordance with the adjusted first treatment mode and/or the second treatment mode.

In still further aspects, a system includes an X-ray tube to emit kilovoltage X-ray radiation towards a portion of a patient in accordance with a treatment plan, a delivery device to deliver a treatment agent to the portion of the patient in accordance with the treatment plan, and a control device to the determine that treatment is not proceeding in accordance with a desired treatment outcome, and in response to the determination. The control device may control the X-ray tube to adjust the emission of the kilovoltage X-ray radiation and/or the delivery device to adjust the delivery of the treatment agent.

The claims are not limited to the disclosed embodiments, however, as those in the art can readily adapt the description herein to create other embodiments and applications.

BRIEF DESCRIPTION OF THE DRAWINGS

The construction and usage of embodiments will become readily apparent from consideration of the following specification as illustrated in the accompanying drawings, in which like reference numerals designate like parts, and wherein:

FIG. 1 is a flow diagram of process steps according to some embodiments;

FIG. 2 is a view of diagnostic computed tomography (CT) equipment;

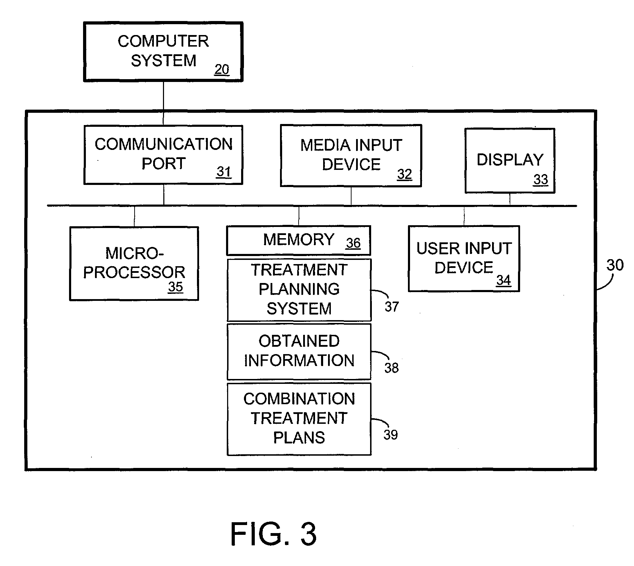

FIG. 3 is a block diagram illustrating elements of diagnostic computed tomography (CT) equipment according to some embodiments;

FIG. 4 is a view of a system to manage combination treatment according to some embodiments;

FIG. 5 is a flow diagram of process steps according to some embodiments;

FIG. 6 is a view of a system to deliver combination treatment according to some embodiments;

FIG. 7 is a block diagram illustrating elements of a system to deliver combination treatment according to some embodiments; and

FIG. 8 is a representative view of a portion of a data table according to some embodiments.

DETAILED DESCRIPTION

The following description is provided to enable any person in the art to make and use the claimed invention and sets forth the best mode contemplated by the inventors for carrying out the claimed invention. Various modifications, however, will remain readily apparent to those in the art.

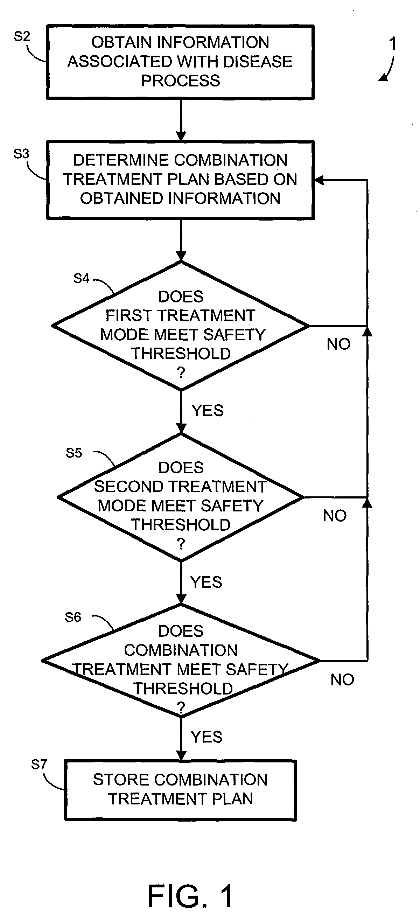

FIG. 1 is a flow diagram of process steps 1 according to some embodiments. Process steps 1 may be implemented, in whole or in part, by hardware and/or executable software. One or more of process steps 1 may be performed manually. Software embodying one or more of process steps 1 may be stored by any medium, including a fixed disk, a floppy disk, a CD-ROM, a DVD-ROM, a Zip™ disk, a magnetic tape, or a signal. Some or all of such software may also be stored in one or more devices.

Process steps 1 may be executed to determine a combination treatment plan and to evaluate a safety of the combination treatment plan. The combination treatment plan may include a first treatment mode and a second treatment mode. According to some embodiments, a treatment agent is present within a portion of the patient according to the first treatment mode and the portion of the patient is irradiated according to the second treatment mode. Some embodiments of process steps 1 provide a safer, more efficient, and/or more effective evaluation of combination treatment than previously available.

Briefly, some embodiments of process steps 1 provide determination of a treatment plan associated with a patient, the treatment plan including a first treatment mode in which a treatment agent is present within a portion of the patient and a second treatment mode in which the portion of the patient is irradiated, and determination, independent from the second treatment mode, of whether the first treatment mode meets a first safety threshold. Such embodiments also include determination, independent from the first treatment mode, of whether the second treatment mode meets a second safety threshold, and determination of whether a combination of the first treatment mode and the second treatment mode meets a third safety threshold.

Initially, information associated with a disease process is obtained at step S2. The information may comprise any information suitable to develop a treatment plan, and may be acquired from or by disparate sources. Some embodiments of S2 include acquisition of medical records and images of an internal volume, or portion, of a patient. Such images may be obtained by computed tomography (CT), magnetic resonance, and/or any other imaging system that is or becomes known.

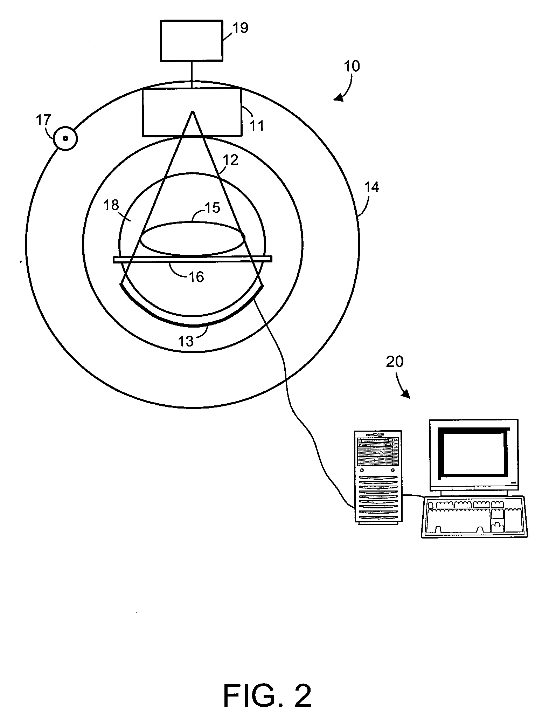

FIG. 2 illustrates a system to acquire an image of a portion of a patient according to some embodiments of step S2. The system comprises CT scanner 10 and computer system 20. CT scanner 10 includes X-ray source 11 for emitting fan-shaped X-ray beam 12 toward radiation receiver 13. Both X-ray source 11 and radiation receiver 13 are mounted on ring 14 such that they may be rotated through 360 degrees while maintaining the physical relationship therebetween.

In general operation, patient 15 is positioned on bed 16 to place a portion of patient 15 between X-ray source 11 and radiation receiver 13. Next, X-ray source 11 and receiver 13 are rotated by rotation drive 17 around cavity 18 in which patient 15 lies. During this rotation, X-ray source 11 is powered by high-voltage generator 19 to transmit X-ray radiation toward receiver 13. Receiver 13 receives the radiation and produces a projection image for each projection angle.

The projection images are transmitted to computer system 20. Computer system 20 calculates attenuation coefficients (e.g., Hounsfield numbers) of predetermined points based on the images. The attenuation coefficients may be used to generate an image representing the portion of patient 15 that lies between X-ray source 11 and radiation receiver 13. The image may be a three-dimensional image, a two-dimensional cross-sectional ("slice") image, or any other type of image ac-

A combination treatment plan is determined at step S3 based on the information obtained at step S2. The combination treatment plan may include a first treatment mode in which a treatment agent is present within a portion of the patient and a second treatment mode in which the portion of the patient is irradiated. According to some embodiments, a radiation oncologist specifies a radiation dose, a target within the patient portion, a radiation beam energy, one or more positions of a radiation delivery device, a type, amount and concentration of treatment agent, a delivery method and rate for the treatment agent, and/or any other currently- or hereafter-known treatment plan parameter. The treatment plan may be formatted as required by a treatment planning system that will be used to execute the treatment plan.

A radiation oncologist or other person may use a treatment planning computer to determine the combination treatment plan at step S3. FIG. 2 is a block diagram of treatment planning computer 30 according to some embodiments. Treatment planning computer 30 includes communication port 31 for receiving images or other information from computer system 20. Communication port 31 may therefore comprise any type of interface suitable for receiving data from computer system 20. Computer 30 also includes media input device that may comprise a CD-ROM drive, a ZIP drive, a USB drive and/or any device for receiving a storage medium and reading data from the medium. The data may include information obtained in step S2.

Display 33 may comprise any one or more devices for displaying images and control interfaces to a user. User input device 34 may be used by the user to input data and commands to computer 30. User input device 34 may comprise any input device or devices that are or become known. A user may utilize display 33 and input device 34 to view information and determine a combination treatment plan.

Microprocessor 35 executes processor-executable process steps stored in memory 36. In this regard, memory 36 stores processor-executable process steps of treatment planning system 37. Steps of treatment planning system 37 may be executed by microprocessor 35 to determine a treatment plan based on the information obtained in step S2, which is stored among obtained information 38. The treatment plan, in turn, is stored among combination treatment plans 39.

Treatment planning system 37 may provide various degrees of treatment planning automation. Generally, treatment planning system 37 may comprise any current or future treatment planning system capable of generating a combination treatment plan that includes a first treatment mode in which a treatment agent is present within a portion of the patient and a second treatment mode in which the portion of the patient is irradiated. One example of treatment planning system 37 according to some embodiments is the KONRAD© treatment planning system of Siemens Corporation©.

Returning to process steps 1, it is determined in step S4 whether the first treatment mode of the determined combination treatment plan meets a first safety threshold. The determination of step S4 is independent of the second treatment mode. That is, according to some embodiments, the determination does not take into account any irradiation specified by the combination treatment plan.

Step S4 may comprise any currently- or hereafter-known system to evaluate a safety threshold with respect to a treatment agent. According to some embodiments, the first treatment mode is associated with the presence of treatment agent within the portion of the patient. The first treatment mode may also specify the type, amount and concentration of the treatment agent as well as the system used to deliver the treatment agent to the portion. In some embodiments of step S4, process steps of treatment planning system 37 are executed to determine the toxicity of the specified agent concentration with respect to the tissue in which the agent is to be delivered. The evaluation may also take into account the diffusion of the agent into surrounding tissues, and the relative risks of the delivery system. Treatment planning system 37 may include any data required to execute step S4, including but not limited to a definition of the first safety threshold and toxicity data for the treatment agent.

If the first safety threshold is met, it is determined in step S5 whether the second treatment mode meets a second safety threshold. The determination of step S5 is independent of the first treatment mode. More particularly, according to some embodiments, the determination of step S5 does not take into account any aspect of the treatment agent or its delivery as specified by the combination treatment plan.

As described above, the second treatment mode includes irradiation of a patient portion. Step S5 may therefore comprise any currently- or hereafter-known system to evaluate a safety threshold with respect to irradiation of a patient portion. In some embodiments, process steps of treatment planning system 37 are executed at step S5 to determine the total dose absorbed by various volumes of the patient according to the treatment plan. Such a determination may comprise Monte Carlo simulations of each radiation beam defined by the treatment plan. In this regard, the treatment plan may call for irradiation of the patient portion by divergent beams originating from different positions external to the patient. The determination may take into account a shape, intensity and energy of each defined radiation beam.

Commonly-assigned U.S. application serial no. 10/252,980, filed September 23, 2002 (Attorney Docket No. 2002P10230US), entitled "Planning System For Convergent Radiation Treatment", provides some other examples for determining a dose delivered to tissues by a convergent radiation beam. One or more of these examples may be employed in some embodiments of step S5.

If the second safety threshold is met, it is then determined whether the combination treatment meets a third safety threshold in step S6. Step S6 may therefore comprise any currently- or hereafter-known system to evaluate a safety threshold with respect to the combination treatment. For example, process steps of treatment planning system 37 may be executed at step S5 to determine the dose enhancement attributable to the treatment agent and the defined beam(s). This dose enhancement may be determined with respect to a target site as well as to other tissues that may receive the treatment agent and be irradiated by the beam(s).

In some embodiments, the dose enhancement of an iodine treatment agent as compare to water may be determined by the equation:

$$[(\square_{en}/\square)I*fx + (1-fx)(\square_{en}/\square)H_2O]/(\square_{en}/\square)H_2O,$$

where fx is the fraction by weight of an iodine treatment agent, and $(\square_{en}/\square$ and $(\square_{en}/\square)H_2O$ are the mass energy absorption coefficients at the effective radiation energy for iodine and water, respectively.

In a second example, the dose enhancement is determined by acquiring a CT image of the portion while the portion is injected with contrast agent at time to. The dose enhancement at a radiation energy between 40 to 80 keV is determined from the Hounsfield Units of the portion read directly from the CT image. CT data of the portion is then periodically acquired as a concentration of contrast agent within the portion changes in accordance with the combination treatment plan.

Dose enhancement values corresponding to each of the periodically-acquired CT data are determined, resulting in a table of various dose enhancement values and corresponding time values > to. The table of values may then be used to determine a dose enhancement function $d/d_0 = f(t)$, wherein d represents, for the particular radiation energy, the dose experienced by a volume using a treatment agent, and do represents the dose experienced by the volume without using a treatment agent. The radiation beam may be diverging and the beam's spectral weighting may be accounted for. Aforementioned U.S. application serial no. 10/252,980, (Attorney Docket No. 2002P10230US) provides other examples for determining a radiation dose received by a patient volume that includes a treatment agent and receives a convergent radiation beam.

Flow returns to step S3 if any of the determinations of steps S4, S5 and S6 are negative. If not, flow continues to step S7 to store the determined treatment plan. The treatment plan may be stored, for example, among combination treatment plans 39 of treatment planning computer 30.

According to some embodiments, first, second and third efficacy thresholds are evaluated at step S4, S5 and S6, respectively. More specifically, step S4 may include a determination of whether the first treatment mode meets a first efficacy threshold, step S5 may include a determination of whether the second treatment mode meets a second efficacy threshold, and/or step S6 may include a determination of whether the third treatment mode meets a third efficacy threshold. The evaluations of the first, second and third efficacy thresholds may be independent of or integrated with the evaluations of their respective safety thresholds.

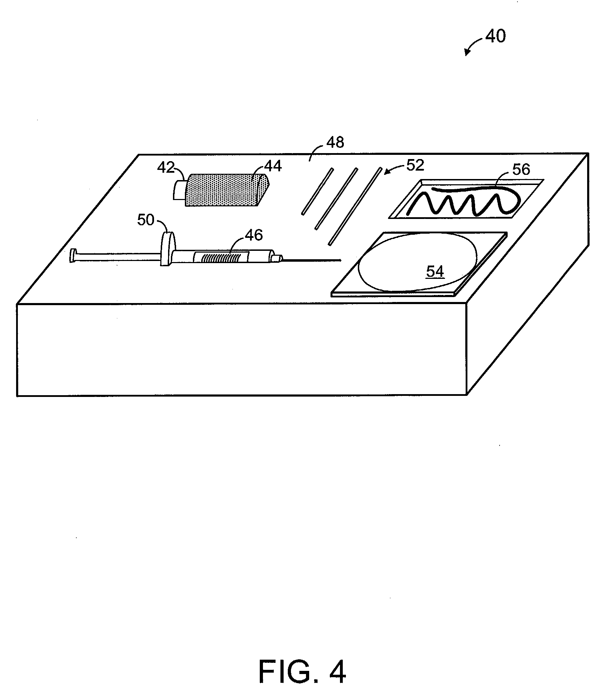

FIG. 4 is a perspective view of a system that may be used in conjunction with some embodiments of process steps 1. System 40 comprises container 42 containing treatment agent 44, and identifier 46 associated with treatment agent 44. According to some embodiments, identifier 46 is usable to identify a treatment plan. As will be described below, the identified treatment plan might be associated with a patient identifier and/or might not be associated with any particular patient.

Treatment agent 44 may comprise any currently- or hereafter-known composition that is capable of treating tissue in response to received radiation. Treatment agent 44 may comprise a heavy element-carrying biochemical agent in some embodiments. Agent 44 according to some embodiments carries one or more of Iodine, Gold, Gadolinium, or other Lanthanide elements, such as Erbium. Leukine may be employed as treatment agent 44, due to its ability to treat tissue in response to received X-ray radiation.

Container 42, agent 44, and identifier 46 are disposed within package 48. Also included in package 48 are syringe 50 on which identifier 46 is disposed, needles 52, software medium 54 and catheter 56. Syringe 50, needles 52 and catheter 56 comprise devices for delivering agent 44 to a patient. One or more of syringe 50, needles 52 and catheter 56 may be particularly suitable to the delivery of a treatment agent having the composition and/or concentration of agent 44.

Software medium 54 may store processor-executable process steps to implement process steps 1. The process steps may be executed by microprocessor 35 to determine a treatment plan associated with a patient, the treatment plan including a first treatment mode in which a treatment agent is present within a portion of the patient and a second treatment mode in which the portion of the patient is irradiated, to determine if, independent from the second treatment mode, the first treatment mode meets a first safety threshold, to determine if, independent from the first treatment mode, the second treatment mode meets a second safety threshold, and to determine if a combination of the first treatment mode and the second treatment mode meets a third safety threshold.

Software medium 54 may also store a combination treatment plan in a computer-readable format. The combination treatment plan may include a first mode in which treatment agent 44 is located in a patient portion, and in which treatment agent 44 is delivered to the patient portion using one or more of syringe 50, needles 52 and catheter 56. The treatment plan may be suitable for evaluation in accordance with steps S4 through S6 using process steps stored on medium 54.

Software medium 54 may also or alternatively store computer-executable process steps to calculate therapeutic effects of treatment agent 54. Such steps may comprise steps to model navigation and localization of a needle in the target, the dissipation of agent 54 within tissue, to calculate a dose enhancement due to the receipt by agent 54 of X-rays having particular energies, or any other steps that may be usable to execute steps S4 through S6.

System 40 may include less or more elements than depicted in FIG. 4. Non-exhaustive examples of such elements include one or more of patient positioning devices, image-guided and other navigation systems, assays, radiation sources, or other treatment agents including nanoparticles. System 40 may also be packaged in suitable manners other than that shown in FIG. 4.

System 40 may be aggregated by an entity that is to deliver radiation treatment, such as a hospital. For example, a radiation oncologist may generate a combination treatment plan based on previously-acquired computed tomography images of a patient and may associate the treatment plan with a patient identifier and identifier 46. System 40 may then be created with elements 42 through 56 that are selected particularly for execution of the treatment plan. Identifier 46 may therefore be used to confirm that system 40 and radiation treatment agent 44 are associated with the generated combination treatment plan prior to delivery of the treatment.

The elements of system 40 may be associated within package 48 by any one or more entities. A manufacturer or reseller of agent 44 may create system 40 and provide system 40 to entities that deliver combination treatment. According to some of these embodiments, software medium 54 may store a combination treatment plan that is not associated with any particular patient. For example, the combination treatment plan may be suitable for particular-sized tumors at particular tissue locations, and the volume and composition of agent 44 (as well as the selection of elements 42 through 54) may be suitable for treating a tumor of the certain size and location. A manufacturer or reseller may also produce system 40 in view of a particular patient.

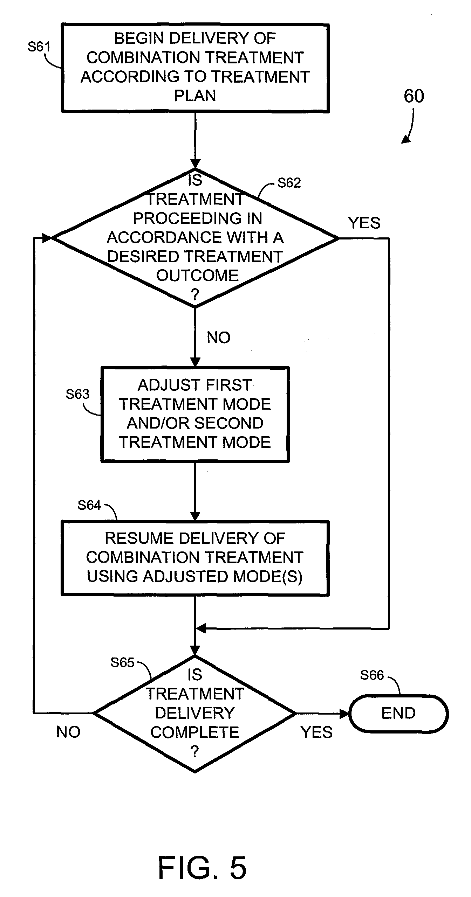

Process steps 60 of FIG. 5 may be used to deliver combination treatment according to some embodiments. Process steps 60 may be implemented, in whole or in part, by hardware and/or software stored by any medium. Some or all of such software may also be stored in one or more devices. One or more of process steps 60 may be performed manually.

Briefly, process steps 60 provide delivery of treatment to a patient in accordance with a first treatment mode in which a treatment agent is present within a portion of the patient and a second treatment mode in which the portion of the patient is irradiated. Also provided are determination that the delivered treatment is not proceeding in accordance with a desired treatment outcome, adjustment of the first treatment mode and/or the second treatment mode, and delivery of treatment to the patient in accordance with the adjusted first treatment mode and/or the second treatment mode. Some embodiments of process steps 60 provide a safer, more efficient, and/or more effective delivery of combination treatment than previously available.

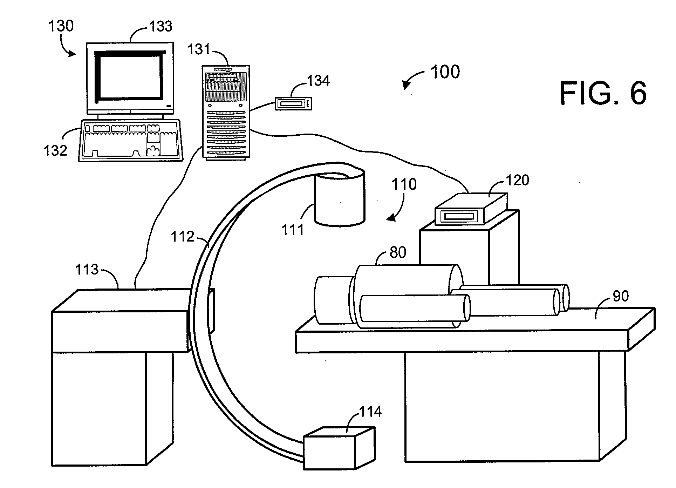

FIG. 6 illustrates elements that may implement process steps 60 according to some embodiments. After the following description of these elements, process steps 60 will be described with respect thereto. Embodiments of process steps 60 are not limited to those described with respect to FIG. 6.

FIG. 6 shows patient 80, table 90 and combination treatment delivery system 100. According to some embodiments, delivery system 100 is used to deliver treatment agent to a portion of a patient and to irradiate the portion according to a combination treatment plan. The environment illustrated in FIG. 6 may include less or more elements than those shown.

Radiation unit 110 of system 100 includes treatment head 111, C-arm 112, base 113 and imaging system 114. In some embodiments, radiation unit 110 comprises a modified CT scanner. Treatment head 111 includes a beam-emitting device such as an X-ray tube for emitting radiation used during calibration, data acquisition and/or treatment. The radiation may comprise electron, photon or any other type of radiation, and may have energies ranging from 50 to 150 keV. The radiation emitted by treatment head 111 may comprise any radiation suitable for data acquisition and/or treatment according to some embodiments. In some embodiments, the radiation is suitable to produce dose-enhancing effects when used in conjunction with a treatment agent that is capable of treating tissue in response to received X-ray radiation.

Treatment head 111 also may include a cylinder in which is disposed a concentrator to concentrate the emitted radiation. The concentrator may comprise optics such as a focusing lens for producing a convergent radiation beam from radiation emitted by the X-ray tube. Examples of this type of lens are described in U.S. Patent No. 6,560,312 to Cash, in U.S. Patent No. 6,359,963 to Cash, in U.S. Patent No. 6,389,100 to Verman et al, in U.S. Patent No. 5,604,782 to Cash, Jr., in U.S. Patent Application Publication No. 2003/0012336 of Cash et al., in U.S. Patent Application Publication No. 2001/0043667 of Antonell et al., and/or elsewhere in currently or hereafter-known art. Treatment head 111 may also include beam-shaping devices such as one or more jaws, collimators, reticles and apertures.

Imaging device 114 may comprise an image intensifier and a camera. An image intensifier is a vacuum tube that converts X-rays to visible light, which is then detected by the camera to produce an image. Imaging device 114 may comprise a flat-panel imaging device using a scintillator layer and solid-state amorphous silicon photodiodes deployed in a two-dimensional array. The RID1640, offered by Perkin-Elmer®, Inc. of Fremont, California, is one suitable device.

Imaging device 114 may comprise other types of imaging devices. For example, imaging device 114 may comprise an amorphous selenium-based device or a CCD or tube-based camera. The latter imaging devices may include a light-proof housing within which are disposed a scintillator, a mirror, and a camera.

Treatment head 111 and imaging device 114 may be coupled to C-arm 112 so as to face one another irrespective of any movement of C-arm 112 with respect to base 113. In this regard, C-arm 112 is slidably mounted on base 113 and can therefore be moved in order to change the position of treatment head 111 with respect to table 90. Table 90 may also be adjustable to assist in positioning an internal portion of patient 80 with respect to radiation unit 110. In some embodiments, base 113 includes a high-voltage generator for supplying power used by treatment head 111 to generate kilovoltage radiation.

Many C-arm/base configurations may be used in conjunction with some embodiments, including portable configurations, configurations in which base 113 is rotatably mounted to a ceiling, configurations in which one C-arm is slidably mounted on another C-arm, and configurations incorporating multiple independent C-arms. Embodiments of radiation unit 110 may comprise one of the SIREMOBIL®, MULTISTAR®, BICOR® and POLYSTAR® systems produced by Siemens Corporation® or other systems designed to emit treatment radiation such as a Siemens Corporation® CT scanner with a Straton® X-ray tube.

In operation, radiation unit 110 may deliver radiation to a portion of patient 80 according to a treatment plan. Radiation unit 110 may be controlled during the delivery of such radiation to change an energy of a radiation beam, an intensity of the radiation beam, a shape of the radiation beam, an angle of the radiation beam, or a point at which the radiation beam enters the patient. Examples of such control will be described below with respect to process steps 60.

Delivery device 120 may comprise any currently- or hereafter-known system for delivering treatment agent to a portion of patient 120. Delivery device 120 may comprise an infusion pump, a catheter and a needle in accordance with some embodiments. Delivery device 120 may execute a program and/or be controlled by operator station 130 to deliver treatment agent according to a combination treatment plan. In some embodiments, delivery device 120 may change and/or be controlled to change a concentration of the treatment agent, a delivery rate of the treatment agent, or a delivery location of the treatment agent. Device 120 may also deliver a second treatment agent to the portion of patient 80.

Operator station 130 includes processor 131 in communication with keyboard 132, display 133 and identifier input device 134. An operator may operate operator station 130 to instruct radiation unit 110 to deliver X-ray radiation and delivery device 120 to deliver treatment agent 44 according to a combination treatment plan stored in processor 131.

Identifier input device 134 may be used to input information such as identifier 46 and/or patient identifiers to operator station 130. Identifier input device 134 may comprise one or more of a smart card scanner, a barcode scanner, a fingerprint scanner, a keypad, or any other input device. Information input by identifier input device 134 may be used to identify a combination treatment plan to be executed. This identification according to some embodiments will be described below.

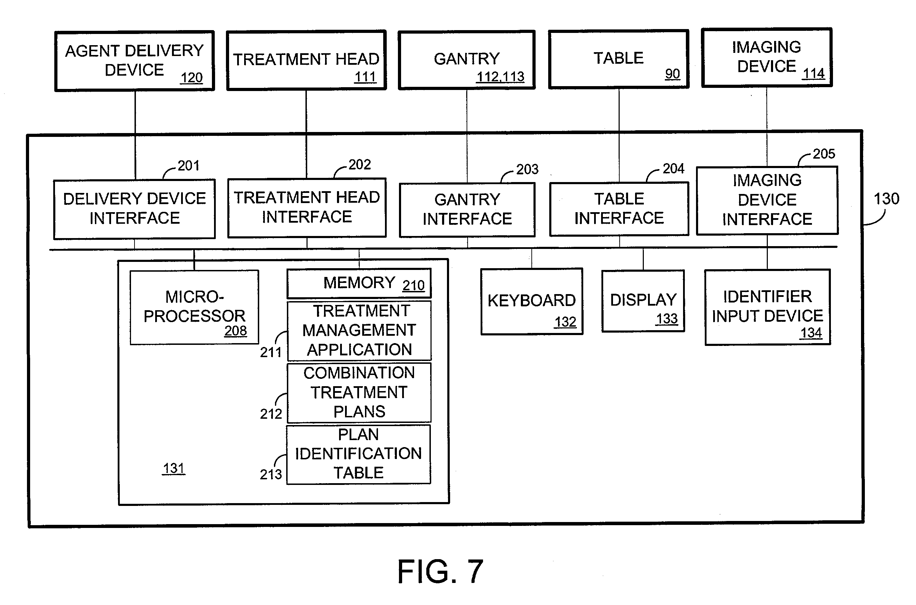

FIG. 7 is a block diagram of elements of FIG. 6 according to some embodiments. As shown, operator station 130 includes several elements for interfacing with other elements of FIG. 6. Specifically, operator station 130 includes delivery device interface 201, treatment head interface 202, gantry interface 203, table interface 204, and imaging device interface 205. Interfaces 201 through 205 may comprise dedicated hardware and/or software interfaces, and one or more of interfaces 201 through 205 may reside in processor 131. One or more of interfaces 201 through 205 may be implemented by a single interface. For example, interfaces 201 and 204 may be implemented by a single Ethernet interface and interfaces 202 through 204 may be implemented by a single proprietary interface for interfacing with table 90, treatment head 111, gantry 112, and base 113.

Processor 131 includes microprocessor 208 and memory 210. Microprocessor 208 executes processor-executable process steps stored in memory 210. In this regard, memory 210 stores processor-executable process steps of treatment management application 211. Treatment management application 211 may comprise processor-executable process steps to implement process steps 60. Memory 210 may also store combination treatment plans 212 and plan identification table 213. Combination treatment plans 212 may comprise scripts that are automatically executable by radiation unit 110, delivery device 120, and treatment table 90 in order to provide combination treatment. Combination treatment plans 212 may also comprise any other currently- or hereafter-known types of combination treatment plan.

Plan identification table 213 may associate identifiers and patient identifiers with combination treatment plans. The identifiers may in turn be associated with treatment agents. In some embodiments, plan identification table 213 may allow for identification of one of combination treatment plans 212 based on a patient identifier and an identifier associated with a treatment agent.

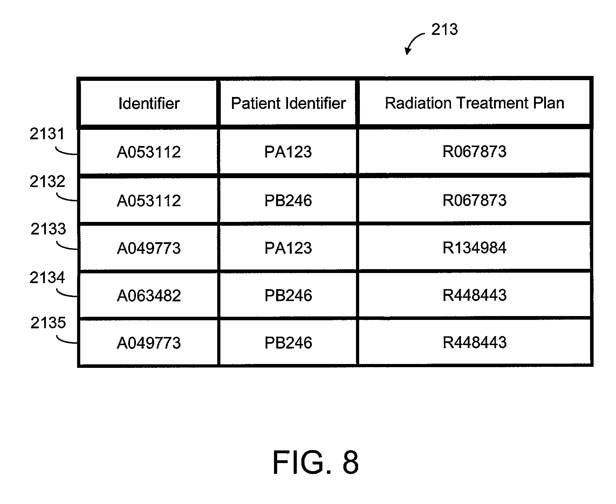

FIG. 8 illustrates a tabular representation of a portion of plan identification table 213 according to some embodiments. Each record of plan identification table 213 associates a combination treatment plan, a patient identifier and an identifier. A combination treatment plan is identified in table 213 by a code that may serve as an index to a combination treatment plan stored among combination treatment plans 212.

Records 2131 and 2132 of table 213 show that a same combination treatment plan and a same radiation treatment agent may be associated with more than one patient. Records 2133 through 2135 show radiation treatment plans that are unique to each patient identified therein. Moreover, records 2134 and 2135 show that a same radiation treatment plan may be associated with more than one radiation treatment agent.

Returning to process steps 60, delivery of combination treatment according to a treatment plan is begun at step S61. In some embodiments, the treatment plan is stored among combination treatment plans 212 and delivery of the combination treatment according to the plan is begun by operator station 130.

For example, operator station 130 may use delivery device interface 201 to control delivery device 120 to deliver treatment agent 44 to a portion of patient 80 according to the treatment plan. Treatment agent 44 may be delivered via direct injection, intravenous injection, or by other means. Operator station 130 may also control treatment head interface 202 to control treatment head 11 so as to implement particular radiation beam parameters called for by the combination treatment plan. These parameters may include an X-ray tube potential, a radiation energy, an X-ray tube current, a scan time and radiation filtration parameters. Moreover, gantry interface 203 and table interface 204 may operate to position treatment head 11 and patient 80 so that the radiation beam is delivered spatially as required by the combination treatment plan.

According to some embodiments, the treatment plan to be executed is determined prior to step S61. For example, a patient identifier associated with patient 80 is first determined. Scanner 134 may read the patient identifier from a patient tag or smart card carried by patient 80. Next, an identifier associated with treatment agent 44 is determined. In some embodiments, the identifier is represented by barcode 46 located on syringe 50. Scanner 134 may determine the identifier by scanning barcode 46.

The patient identifier and the identifier are then used to identify a combination treatment plan. Accordingly, the identifier and the patient identifier may be used in conjunction with plan identification table 213 to identify a combination treatment plan from among treatment plans 212.

The patient identifier may comprise any perceptible article capable of identifying patient 80. Additionally, any suitable system may be used to associate agent 44 with an identifier. As non-exhaustive examples, the identifier may be placed on a container containing agent 44 such as container 42, on a package containing agent 44 and related elements such as package 48, or on a surgical tray holding agent 44 and delivery devices used to deliver the agent.

At step S62, it is determined whether the delivery of treatment is proceeding in accordance with a desired treatment outcome. The desired treatment outcome may comprise one or more of a desired dose distribution, a desired beam shape or energy, a desired distribution of treatment agent 44, a desired concentration of treatment agent 44, a desired diffusion rate of treatment agent 44, or any other outcome. The determination of step S62 may be based on one or more of images captured by imaging device 114 under the control of imaging device interface 205, radiochromic film or dosimeter readings, cone beam-reconstructed images of patient 80 during captured by radiation unit 110 during delivery of the combination treatment, feedback received from delivery device 120 via delivery device interface 201, Monte Carlo simulations, a detected concentration of treatment agent 44 within a region of interest of patient 80, or any other indicator of treatment outcome.

Commonly-assigned U.S. application serial no. (2002P12620US) describes one method to determine a concentration of treatment agent 44 within patient 80. Generally, a CT image of the region of interest is acquired and a CT number corresponding to the region of interest is determined from the acquired CT image. A pre-populated calibration table is identified that corresponds to the scanning parameters used to acquire the CT image, and a concentration associated with the CT number is determined from the table. Interpolation may be used in a case that the scanning parameters or the CT number is not identically specified in one of calibration tables.

If the determination in step S62 is negative, the first treatment mode and/or the second treatment mode are adjusted in step S63. Adjustment of the first treatment mode may include one or more of controlling delivery device 120 via delivery device interface 201 to change a concentration of treatment agent 44, a delivery rate of treatment agent 44, or a delivery location of treatment agent 44. Device 120 may also be controlled to deliver a second treatment agent to the portion of patient 80. The concentration of treatment agent 44 may also be changed by waiting for patient 80's biological processes to flush out a portion of agent 44 from the region of interest. Any other adjustment related to the presence of treatment agent 44 within patient 80 may be executed at step S63.

Adjustment of the second treatment mode may include one or more of controlling radiation unit 110 via interfaces 202 through 204 to change an energy of the radiation beam, an intensity of the radiation beam, a shape of the radiation beam, an angle of the radiation beam, a point at which the radiation beam enters patient 80, or any other desired irradiation parameter. Flow then proceeds to step S64 of process steps 60.

Delivery of the combination treatment resumes at step S64 using the adjusted first treatment mode and/or second treatment mode. Next, at step S65, it is determined whether the treatment delivery is complete. Such a determination may be based on the combination treatment plan and/or a desired outcome of the combination treatment plan. For example, treatment delivery may be deemed complete if all steps of the combination treatment plan have been executed and/or if a desired dose distribution has been delivered. As shown in FIG. 5, flow also proceeds directly to step S65 if the determination at step S62 is positive.

Process steps 60 terminate at step S66 if it is determined that treatment delivery is complete. If treatment delivery is not complete, flow returns to step S62 and continues as described above. According to some embodiments, an assessment of treatment is performed subsequent to process steps 60 and flow returns to step S62 if the assessment is found unsatisfactory.

Those in the art will appreciate that various adaptations and modifications of the above-described embodiments can be configured without departing from the scope and spirit of the claims. Therefore, it is to be understood that the claims may be practiced other than as specifically described herein.

**Claims**

1. A method comprising:

   determining (S3) a treatment plan associated with a patient, the treatment plan comprising a first treatment mode in which a treatment agent is present within a portion of the patient and a second treatment mode in which the portion of the patient is irradiated;
   determining (S4) if, independent from the second treatment mode, the first treatment mode meets a first safety threshold;
   determining (S5) if, independent from the first treatment mode, the second treatment mode meets a second safety threshold; and
   determining (56) if a combination of the first treatment mode and the second treatment mode meets a third safety threshold.

2. A method according to Claim 1, wherein the treatment agent comprises a radiation dose-enhancing agent (44), and
   wherein the second treatment mode comprises delivering X-ray radiation to the portion of the patient.

3. A method according to Claim 2, wherein the X-ray radiation comprises focused kilovoltage X-ray radiation.

4. A method according to Claim 1, wherein determining if the first treatment mode meets the first safety threshold comprises determining a toxicity of the treatment agent with respect to the portion of the patient.

5. A method according to Claim 4, wherein determining if the second treatment mode meets the second safety threshold comprises determining an effect of one or more radiation beams on the patient.

6. A method according to Claim 5, wherein determining if the combination meets the third safety threshold comprises evaluating an interaction between the treatment agent, the one or more radiation beams, and the patient specified by the treatment plan.

7. A method according to Claim 1, wherein determining if the combination meets the third safety threshold comprises evaluating an interaction between the treatment agent, the one or more radiation beams, and the patient specified by the treatment plan.

8. A method according to Claim 1, further comprising:

   delivering (S61) treatment to the patient in accordance with the first treatment mode and the second treatment mode;
   determining (S62) that the delivered treatment is not proceeding in accordance with a desired treatment outcome;
   adjusting (S63) the first treatment mode and/or the second treatment mode; and
   delivering (S64) treatment to the patient in accordance with the adjusted first treatment mode and/or the second treatment mode.

9. A method according to Claim 8, wherein adjusting the first treatment mode and/or the second treatment mode comprises at least one of:

   changing a concentration of the treatment agent; changing a delivery rate of the treatment agent; changing a delivery location of the treatment agent; or delivering a second treatment agent to the portion of the patient.

10. A method according to Claim 9, wherein adjusting the first treatment mode and/or the second treatment mode comprises at least one of:

    changing an energy of a radiation beam; changing an intensity of the radiation beam; changing a shape of the radiation beam; changing an angle of the radiation beam; or changing a point at which the radiation beam enters the patient.

**11.** A method according to Claim 8, wherein adjusting the first treatment mode and/or the second treatment mode comprises at least one of:

changing an energy of a radiation beam; changing an intensity of the radiation beam; changing a shape of the radiation beam; changing an angle of the radiation beam; or changing a point at which the radiation beam enters the patient.

**12.** A method according to Claim 8, further comprising, prior to delivering the treatment to the patient,:

associating an identifier with the treatment plan and the treatment agent;
determining the identifier associated with the treatment agent;
determining the patient identifier; and
determining the radiation treatment plan associated with the identifier and the patient.

**13.** A method comprising:

delivering (S61) treatment to a patient in accordance with a first treatment mode in which a treatment agent is present within a portion of the patient and a second treatment mode in which the portion of the patient is irradiated;
determining (S62) that the delivered treatment is not proceeding in accordance with a desired treatment outcome;
adjusting (S63) the first treatment mode and/or the second treatment mode; and
delivering (S64) treatment to the patient in accordance with the adjusted first treatment mode and/or the second treatment mode.

**14.** A method according to Claim 13, wherein adjusting the first treatment mode and/or the second treatment mode comprises at least one of:

changing a concentration of the treatment agent; changing a delivery rate of the treatment agent; changing a delivery location of the treatment agent; or delivering a second treatment agent to the portion of the patient.

**15.** A method according to Claim 13, wherein adjusting the first treatment mode and/or the second treatment mode comprises at least one of:

changing an energy of a radiation beam; changing an intensity of the radiation beam; changing a shape of the radiation beam; changing an angle of the radiation beam; or changing a point at which the radiation beam enters the patient.

**16.** A system comprising:

an X-ray tube (111) to emit kilovoltage X-ray radiation towards a portion of a patient in accordance with a treatment plan;
a delivery device (120) to deliver a treatment agent to the portion of the patient in accordance with the treatment plan; and
a control device (130) to the determine that treatment is not proceeding in accordance with a desired treatment outcome, and in response to the determination, the control device to control the X-ray tube to adjust the emission of the kilovoltage X-ray radiation and/or the delivery device to adjust the delivery of the treatment agent.

**17.** A system according to Claim 16, further comprising:

a concentrator (111) located between the X-ray tube and the patient, the concentrator to receive the X-ray radiation and to concentrate the X-ray radiation, and

wherein, in response to the determination, the control device is to control the concentrator to adjust the concentration of the X-ray radiation.

FIG. 1

FIG. 2

EP 1 541 196 A1

```
                    ┌─────────────────┐
                    │    COMPUTER     │
                    │    SYSTEM   20  │
                    └────────┬────────┘
                             │
┌────────────────────────────┼─────────────────────────────────────┐
│   ┌──────────────────┐  ┌──┴──────────────┐  ┌───────────────┐    │
│   │  COMMUNICATION   │  │  MEDIA INPUT    │  │   DISPLAY     │    │
│   │  PORT       31   │  │  DEVICE     32  │  │          33   │    │
│   └────────┬─────────┘  └────────┬────────┘  └───────┬───────┘    │
│            │                     │                   │            │
│   ─────────┴─────────────────────┴───────────────────┴─────────   │
│            │            ┌──────────────────┐         │            │
│   ┌────────┴─────────┐  │  MEMORY   36     │  ┌──────┴────────┐   │
│   │    MICRO-        │  ├──────────────────┤  │  USER INPUT   │   │
│   │    PROCESSOR     │  │  TREATMENT       │  │  DEVICE       │   │
│   │             35   │  │  PLANNING        │  │          34   │ 30│
│   └──────────────────┘  │  SYSTEM      37  │  └───────────────┘   │
│                         ├──────────────────┤                      │
│                         │  OBTAINED        │                      │
│                         │  INFORMATION  38 │                      │
│                         ├──────────────────┤                      │
│                         │  COMBINATION     │                      │
│                         │  TREATMENT       │                      │
│                         │  PLANS       39  │                      │
│                         └──────────────────┘                      │
└───────────────────────────────────────────────────────────────────┘
```

# FIG. 3

FIG. 4

S61 — BEGIN DELIVERY OF COMBINATION TREATMENT ACCORDING TO TREATMENT PLAN

60

S62 — IS TREATMENT PROCEEDING IN ACCORDANCE WITH A DESIRED TREATMENT OUTCOME ?

YES

NO

S63 — ADJUST FIRST TREATMENT MODE AND/OR SECOND TREATMENT MODE

S64 — RESUME DELIVERY OF COMBINATION TREATMENT USING ADJUSTED MODE(S)

S65 — IS TREATMENT DELIVERY COMPLETE ?

NO

YES

S66 — END

FIG. 5

FIG. 6

EP 1 541 196 A1

FIG. 7

EP 1 541 196 A1

EP 1 541 196 A1

213

| Identifier | Patient Identifier | Radiation Treatment Plan |
|---|---|---|
| A053112 | PA123 | R067873 |
| A053112 | PB246 | R067873 |
| A049773 | PA123 | R134984 |
| A063482 | PB246 | R448443 |
| A049773 | PB246 | R448443 |

FIG. 8

**European Patent Office** **PARTIAL EUROPEAN SEARCH REPORT** Application Number

which under Rule 45 of the European Patent Convention EP 04 07 8362
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 2003/206611 A1 (COLLINS WILLIAM F) 6 November 2003 (2003-11-06) * claims 1,4,5 * ----- | 1-7 | A61N5/10 |
| X | US 6 125 295 A (CASH, JR. ET AL) 26 September 2000 (2000-09-26) * column 11, line 58 - column 12, line 48; figure 15 * * column 7, line 31 - line 52 * | 16,17 | |
| Y | ----- | 1-7 | |
| A | US 2002/051513 A1 (PUGACHEV ANDREI ET AL) 2 May 2002 (2002-05-02) * abstract * * paragraph [0018] * ----- | 1,5 | |
| A | EP 1 195 177 A (KUMAKHOV, MURADIN ABUBEKIROVICH) 10 April 2002 (2002-04-10) * paragraph [0083] - paragraph [0086] * ----- | 16 | |

TECHNICAL FIELDS SEARCHED (Int.Cl.7)

A61N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 23 March 2005 | Petter, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C07)

**European Patent
Office**

**INCOMPLETE SEARCH
SHEET C**

**Application Number**

EP 04 07 8362

```
Claim(s) searched completely:
      1-7, 16, 17

Claim(s) not searched:
      8-15

Reason for the limitation of the search (non-patentable invention(s)):

Article 52 (4) EPC - Method for treatment of the human or animal body by
therapy
```

**ANNEX TO THE EUROPEAN SEARCH REPORT
ON EUROPEAN PATENT APPLICATION NO.**

EP 04 07 8362

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2005

| Patent document cited in search report | | Publication date | Patent family member(s) | | Publication date |
|---|---|---|---|---|---|
| US 2003206611 | A1 | 06-11-2003 | WO | 2004026403 A1 | 01-04-2004 |
| | | | US | 2003206610 A1 | 06-11-2003 |
| | | | US | 2003206613 A1 | 06-11-2003 |
| | | | US | 2003206612 A1 | 06-11-2003 |
| US 6125295 | A | 26-09-2000 | AU | 5693499 A | 21-03-2000 |
| | | | EP | 1109489 A1 | 27-06-2001 |
| | | | WO | 0012006 A1 | 09-03-2000 |
| | | | US | 2004006254 A1 | 08-01-2004 |
| | | | US | 6366801 B1 | 02-04-2002 |
| US 2002051513 | A1 | 02-05-2002 | NONE | | |
| EP 1195177 | A | 10-04-2002 | WO | 0202188 A1 | 10-01-2002 |
| | | | AU | 767922 B2 | 27-11-2003 |
| | | | AU | 7563600 A | 14-01-2002 |
| | | | CA | 2384171 A1 | 10-01-2002 |
| | | | EP | 1195177 A1 | 10-04-2002 |
| | | | JP | 2004501730 T | 22-01-2004 |

EPO FORM P0459

For more details about this annex : see Official Journal of the European Patent Office, No. 12/82